(19) Europäisches Patentamt
European Patent Office
Office européen des brevets

(11) Publication number: **0 132 128**
**B1**

(12) # EUROPEAN PATENT SPECIFICATION

(45) Date of publication of patent specification: **09.12.87**

(51) Int. Cl.⁴: **D 21 C 9/00** // A61L15/00

(21) Application number: **84304783.8**

(22) Date of filing: **13.07.84**

(54) **Process for making pulp sheets containing debonding agents.**

(30) Priority: **14.07.83 US 513875**

(43) Date of publication of application:
**23.01.85 Bulletin 85/04**

(45) Publication of the grant of the patent:
**09.12.87 Bulletin 87/50**

(84) Designated Contracting States:
**DE FR SE**

(56) References cited:
**DE-A-3 119 907**
**US-A-3 556 931**
**US-A-3 591 450**
**US-A-4 144 122**

**TAPPI JOURNAL OF THE TECHNICAL ASSOCIATION OF THE PULP AND PAPER INDUSTRY, vol. 65, no. 7, July 1982, pages 93-97, Atlanta, GA., US; J.H. FIELD: "Pulp parameters affecting product performance"**

**ABSTRACT BULLETIN OF THE INSTITUTE OF PAPER CHEMISTRY, vol. 52, no. 5, November 1981, page 533, no. 4889, Appleton, Wisc., US; I.A. STRELYUGINA et al.: "Use of surfactants in manufacture of cordgrade pulp"**

(73) Proprietor: **THE PROCTER & GAMBLE COMPANY**
**301 East Sixth Street**
**Cincinnati Ohio 45202 (US)**

(72) Inventor: **Moran, Richard Thomas**
**6303 Knollcrest Ct.,**
**West Chester Ohio 45069 (US)**
Inventor: **Osborn III, Thomas Ward**
**400 Deanview Drive**
**Cincinnati Ohio 45224 (US)**

(74) Representative: **Gibson, Tony Nicholas et al**
**Procter & Gamble (NTC) Limited Whitley Road**
**Longbenton Newcastle upon Tyne NE12 9TS (GB)**

(56) References cited:
**RESEARCH DISCLOSURE, no. 208, August 1981, page 324, disclosure no. 20836, Havant, Hampshire, GB; "Resin treated cellulosic fibers and process for their manufacture"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 132 128 B1

Courier Press, Leamington Spa, England.

**Description**

Background information

This invention relates to a process for making pulp sheets that contain debonding agents. The debonding agents render the pulp sheets easily fiberizable to form a fluffed fibrous wood pulp batt. The fluffed fibrous wood pulp batt is utilized in the construction of diapers, sanitary napkins, bandages, nursing pads, etc.

It is well known to utilize debonding agents in the formation of pulp sheets in order to render such sheets easily fiberizable. U.S. Patent Re. 26,939 discloses a method for utilizing cationic debonding agents in pulp sheets in order to render such sheets more easily fiberizable. It is stated that the debonding agent must be added to the wet slurry of wood pulp which is then formed into the pulp sheet and subsequently dried. Such pulp sheet can be fiberized to form fluffed fibrous wood pulp batt.

U.S. Patent Nos. 3,554,862 & 3,554,863 disclose a method of forming a pulp sheet consisting of impregnating the pulp with about 0.1% to 5%, based on the weight of the dry weight of the pulp, of a cationic long chain fatty alkyl compound debonding agent having at least 12 carbon atoms in at least one alkyl chain. It is stated that the debonding agent may be added to the pulp or pulp sheet in a number of different places in the pulp sheet formation process. For example, the debonding agent can be added to the pulp slurry in a stock chest, at the head box of the paper machine or at the wet press section of the paper machine. It is stated that the introduction of the debonding agent at the wet press section of the paper machine results in introducing the debonding agent into the previously formed pulp sheet while it is still relatively wet and before the pulp sheet is finally dried on the machine.

J. H. Field in Tappi *65* 1982 July No. 7 page 95 discloses that the effectiveness of nonionic and anionic surfactants as debonding agents for pulp fibre sheets is increased if the surfactants are sprayed on to the pulp during drying rather than being added to a pulp slurry. The effectiveness of cationic surfactants is, in contrast, reduced if they are sprayed on rather than being applied in other ways. This reference is silent as to the moisture content of the sheet when the spray of surfactant is applied and also as to the subsequent treatment of the sheet after spraying.

Thus the present invention permits uniform diffusion of the debonding agent and hence satisfactory fiberisation of the sheet if the more efficient addition of the debonding agent to the formed sheet is adopted.

Summary of the invention

According to the present invention there is provided a process of making fluffed fibrous pulp sheets that contain debonding agents comprising the steps of:

(a) forming said pulp sheet; and

(b) drying said pulp sheet and applying a debonding agent selected from cationic, nonionic and anionic surfactants and mixtures thereof to said pulp sheet, in an amount of from 0.001% to 5.0% based on the dry weight of the pulp sheet, after the commencement of said drying step, wherein the process comprises:

1) applying said debonding agent uniformly to the surface of said pulp sheet after the sheet has been dried to a moisture content of from 5% to 15% by weight of the sheet,
2) storing said treated sheet to permit said debonding agent to diffuse uniformly through said pulp sheet before said sheet is fiberized.

Preferably the debonding agent is an aqueous solution of a cationic surfactant, the solution is sprayed on to the sheet and the treated sheet is held for at least one week before fiberization.

Detailed description of the invention

This invention relates to a process of making a pulp sheet that contains a debonding agent. The process consists of forming a pulp sheet and then applying a debonding agent to the pulp sheet. Experimental evidence indicates that applying the debonding agent to such pulp sheet renders the pulp sheet easily fiberizable. In fact, the pulp sheet made by the process of the present invention is rendered essentially as easily fiberizable as a pulp sheet that contains a debonding agent made by conventional processes, i.e. for example, adding the debonding agent to a wet slurry of wood pulp. The pulp sheet made by the process of the present invention can be fiberized by conventional means such as with a hammermill or other suitable mechanical means and then formed into a fluffed fibrous wood pulp batt.

Numerous benefits flow from applying the debonding agent to the formed pulp sheet. Such application of the debonding agent directly to the pulp sheet is extremely efficient; essentially all of the debonding agent is applied to and utilized by the pulp sheet. The addition of the debonding agent to, for example, the wet slurry of wood pulp does not result in all of the debonding agent being absorbed by the cellulose and, thus, being part of the pulp sheet that is formed. This results in more than just wasting some of the debonding agent. For example, such wasted debonding agent can corrode the paper machine and the attendant equipment, contaminate pulp that will be reusing the water of the pulp slurry and slow drainage of water in the water removal steps of the pulp sheet formation process. All of these inimical effects are avoided by the process of the present invention. Also, the addition of the debonding agent to the

wood pulp slurry results in the debonding agent adsorbing contaminants in the slurry which, when the debonding agents adsorb to the cellulose, deposit the contaminants onto the cellulose. This results in the pulp being of poorer color than that of untreated pulp of the same type. This problem is also avoided by the process of the present invention.

The process of the present invention comprises forming a pulp sheet and then applying a debonding agent to the pulp sheet. Such process can be initiated by utilizing a pulping operation which reduces wood into pulp to form wet slurry of wood pulp.

The next step of the process is the formation of the pulp sheet. The pulp sheet is made from the wood pulp slurry and formation can be carried out on a conventional paper machine of the cylinder or Fourdrinier type. However, any of the methods used by those skilled in the papermaking art to prepare the pulp sheet can be used herein. Typically, the wood pulp slurry is deposited on a foraminous surface, such as a Fourdrinier wire, and water is removed from the furnish by gravity or a vacuum assisted drainage, with or without pressing.

The next step of the process is the drying of the pulp sheet. At the start of the drying step, the pulp sheet generally contains less than 50% by weight of moisture. Any of the techniques well known to those skilled in the papermaking art for drying the pulp sheet can be used. Typically, the pulp sheet is dried by heat supplied by air moving around, over, or through the pulp sheet; by contact with a heated surface; or by a combination of the two methods. However, it should be noted that for the purposes of this invention, any method that removes moisture from the formed pulp sheet constitutes drying of the pulp sheet. The dried pulp sheet contains from 5% to 15% of moisture. The desired level of moisture in the dried pulp sheet is dependent upon the end use of the pulp sheet.

The debonding agent is applied after the pulp sheet has been dried to a level of from 5% to 15% of moisture.

At this stage the dried pulp sheet is divorced from the paper machine. Thus, one can carry out the process of the invention without altering the current paper making process. Also, the application of the debonding agent at this stage prevents the debonding agent from coming into any contact with the paper machine. This eliminates the possibility of the debonding agent corroding the paper machine, albeit the application of the debonding agent to the pulp sheet during the drying step results in less corrosion than when the debonding agent is applied to the wood pulp slurry. Another benefit is that it is possible to make both treated and untreated pulp sheet simultaneously on a single paper machine.

The debonding agent can be applied to the pulp sheet by any of the common techniques well known to those skilled in the art and irrespective of the application technique employed, it is believed that the debonding agent diffuses in time throughout the pulp sheet. However the rapidity of the diffusion is enhanced if the debonding agent is applied substantially evenly and completely onto the pulp sheet surface or surfaces. One suitable and preferred technique of such application is the spraying of the debonding agent onto the pulp sheet surface or surfaces. Depending upon the specific debonding agent used, it may be sprayed onto the pulp sheet surface in its liquid (or molten) state or it may be sprayed onto the pulp sheet surface either dissolved or dispersed in a carrier. Naturally, the specific carrier will depend upon the specific debonding agent used. As a practical matter, water is the preferred carrier for cost and process considerations. In certain circumstances, it may be desirable to apply such a large quantity of carrier to the pulp sheet that a subsequent process operation to remove the carrier from the dried pulp sheet (i.e., to redry the pulp sheet) may be necessary. Any technique commonly used for drying a pulp sheet can be used.

Alternatively, the debonding agent can be applied to the pulp sheet by a padding technique. Here, the debonding agent is usually dispersed or dissolved in a carrier such as water and then the pulp sheet is passed through a bath of the dissolved or dispersed debonding agent. Subsequent drying operations are frequently required when a padding technique is used.

Another alternative method of applying the debonding agent to the pulp sheet involves the use of transfer rolls to convey the debonding agent, usually dissolved or dispersed in a carrier, from a source of supply to the pulp sheet in a manner analogous to printing.

Yet another alternative method of applying the debonding agent to the pulp sheet is by vaporizing the debonding agent in a closed chamber and then passing the pulp sheet through the chamber. This results in the debonding agent depositing onto the pulp sheet.

In a still further alternative method of application, the debonding agent, optionally dispersed is dissolved in a carrier, is extruded directly onto the pulp sheet surface.

Following the application of the debonding agent to the pulp sheet the pulp sheet formed by this invention is ready to be fiberized after the debonding agent has been given a sufficient period of time to diffuse throughout the pulp sheet. This time period is commonly known as aging. As pointed out above, the preferred aging period depends upon where and how the debonding agent is applied to the pulp sheet. Experimental evidence indicates that if the debonding agent was sprayed onto a single pulp sheet surface, it took about one week for the debonding agent to diffuse throughout the pulp sheet which resulted in the pulp sheet being very easily fiberizable.

The level of debonding agent for use in the present invention is dependent upon the nature of the debonding agent selected, the nature of the paper making fibers comprising the pulp sheet and the properties desired in the fluffed fibrous wood pulp batt. Typically, the level of debonding agent is from

0.001% to 5% and more preferably from 0.03% to 0.3% based upon the dry weight of the pulp sheet. The preferred level of debonding agent is heavily dependent upon the end use of the pulp sheet.

Suitable debonding agents for use in the present invention are cationic surfactants which include quaternary ammonium compounds.

Preferred quaternary ammonium compounds include those having the structure:

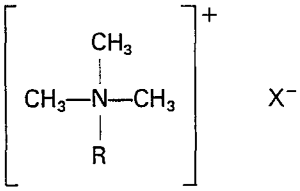

and those having the structure:

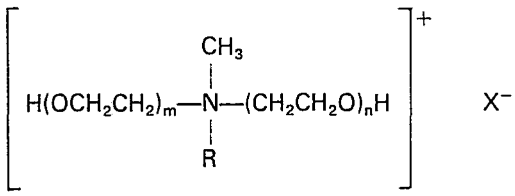

In the two structures noted above R is an aliphatic hydrocarbon radical preferably selected from alkyl radicals having from 12 to 18 carbon atoms, alkylene radicals having from 12 to 18 carbon atoms, coconut and tallow: m and n are both integers each having a value of at least 1; the sum of m and n preferably is from 2 to 15; and X is a halogen.

As used above, "coconut" refers to the alkyl and alkylene moieties derived from coconut oil. It is recognized that coconut oil is a naturally occurring mixture having, as do all naturally occurring materials, a range of compositions. Coconut oil contains primarily fatty acids (from which the alkyl and alkylene moieties of the quaternary ammonium salts are derived) having from 12 to 16 carbon atoms, although fatty acids having fewer and more carbon atoms are also present. Swern, Ed. in *Bailey's Industrial Oil And Fat Products,* Third Edition, John Wiley and Sons (New York; 1964) in Table 6.5, suggests that coconut oil typically has from 65 to 82% by weight of its fatty acids in the 12 to 16 carbon atoms range with about 8% of the total fatty acid content being present as unsaturated molecules. The principle unsaturated fatty acid in coconut oil is oleic acid. Synthetic as well as naturally occurring "coconut" mixtures fall within the scope of this invention.

Tallow, as is coconut, is a naturally occurring material having a variable composition. Table 6.13 in the above-identified reference edited by Swern indicates that typically 78% or more of the fatty acids of tallow contain 16 or 18 carbon atoms. Typically, half of the fatty acids present in tallow are unsaturated, primarily in the form of oleic acid. Synthetic as well as natural "tallows" fall within the scope of the present invention.

Alkylenes are generally preferred to alkyls, Coconut is more preferred than the alkyl and alkylene radicals noted above.

In the case of the methylpolyoxyethylene quaternary ammonium compounds, the sum of m and n is preferably about 2.

Any of the halide salts can be used in the present invention. Typically, and preferably, the chloride is used. Hereinafter quaternary ammonium compounds will frequently be referred to as chlorides for convenience even though the other halide salts are expressly not disclaimed.

Specific examples of quaternary ammonium salts useful in this invention include:

trimethyloctadecylammonium chloride,
trimethylcocoammonium chloride,
trimethyltallowammonium chloride,
trimethylolelylammonium chloride,
methylbis(2-hydroxyethyl)cocoammonium chloride,
methylbis(2-hydroxyethyl)oleylammonium chloride,
methylbis(2-hydroxyethyl)octadecylammonium chloride,
methylbis(2-hydroxyethyl)tallowammonium chloride,
methylpolyoxyethylene(15)cocoammonium chloride, and
methylpolyoxyethylene(15)olylammonium chloride.

These quaternary ammonium compounds can be prepared by any of the means well known to those skilled in the art.

The most preferred quaternary ammonium compound is methylbis(2-hydroxyethyl)cocoammonium chloride. This particular material is available commercially from Armak Company of Chicago, Illinois under the tradename "Ethoquad C/12".

Other cationic debonding agents useful herein are described in U.S. Patent 4,144,122 Emanuelsson et al (March 13, 1979).

These bis-(alkoxy-2(hydroxy)propylene quaternary ammonium compounds have the general formula:

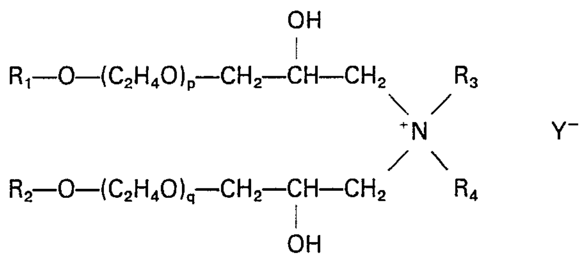

wherein $R_1$ and $R_2$ are aliphatic hydrocarbon groups, either saturated or unsaturated, having from about eight to about twenty-two carbon atoms; $R_3$ and $R_4$ are each selected from methyl, ethyl, and hydroxyethyl; p and q are integers each having a value of from 2 to 20; and $Y^-$ is a salt forming anion and can be either organic or inorganic. Materials such as these are sold under the tradename "Berocell" by Berol Chemie AB of Sweden. The preferred debonding agents are of the above general formula wherein $R_1$ and $R_2$ are dinonyl phenyl and p and q are 14. The specific debonding agent is sold under the tradename Berocell 579.

Another major benefit of the addition of the debonding agent to the formed pulp sheet is that a very wide range of compounds can be utilized as debonding agents. Heretofore, essentially only cationic debonding agents could be efficiently utilized. This is based upon their affinity for the cellulose fibers which carry a negative charge. If a non-cationic debonding agent were utilized, it had to be utilized in combination with a cationic retention agent. For example, U.S. Patent 4,303,471, Laursen (December 1, 1981) discloses nonionic fatty acids esters as debonding agents, but such debonding agents must be utilized in combination with a cationic retention agent such as a cationic surfactant. Since the process of the present invention directly applies the debonding agent to the pulp sheet, a retention agent is not necessary. A very wide range of compounds can be utilized as debonding agents without the use of a cationic retention agent. Such other suitable debonding agents are nonionic and anionic surfactants. Thus, a second aspect of the present invention is a pulp sheet comprising nonionic surfactants, anionic surfactants and mixtures thereof which is substantially free of cationic surfactants. "Substantially free" of cationic surfactants means that the pulp sheet contains less than 0.01% and preferably less than about 0.001% cationic surfactant based upon the dry weight of the pulp sheet.

It is believed that essentially any nonionic surfactant that is applied to the pulp sheet will render it easily fiberizable. Nonlimiting examples of suitable nonionic surfactants are as follows;

(1) The polyethylene oxide condensates of alkyl phenols. These compounds include the condensation products of alkyl phenols having an alkyl group containing from 6 to 12 carbon atoms in either a straight chain or branched chain configuration with ethylene oxide, said ethylene oxide being present in an amount equal to 5 to 25 moles of ethylene oxide per mole of alkyl phenol. The alkyl substituent in such compounds can be derived, for example, from polymerized propylene, diisobutylene, and the like. Examples of compounds of this type include nonyl phenol condensed with 9.5 moles of ethylene oxide per mole of nonyl phenol; dodecylphenol condensed with 12 moles of ethylene oxide per mole of phenol; dinonyl phenol condensed with 15 moles of ethylene oxide per mole of phenol; and diisooctyl phenol condensed with 15 moles of ethylene oxide per mole of phenol. Commercially available nonionic surfactants of this type include Igepal CO-630, (Igepal is a Registered Trade Mark) marketed by the GAF Corporation, and Triton X-45, X-114, X-100, and X-102, all marketed by the Rohm and Haas Company.

(2) The condensation products of aliphatic alcohols or aliphatic fatty acid with from 1 to 25 moles of ethylene oxide. The alkyl chain of the aliphatic alcohol or aliphatic fatty acid can either be straight or branched, primary or secondary, and generally contains from 8 to 22 carbon atoms. Examples of such ethoxylated alcohols include the condensation product of myristyl alcohol condensed with 10 moles of ethylene oxide per mole of alcohol; and the condensation product of 9 moles of ethylene oxide with coconut alcohol (a mixture of fatty alcohols with alkyl chains varying in length from 10 to 14 carbon atoms). Examples of commercially available nonionic surfactants in this type include Pegosperse 200 ML marketed by Glyco Inc., Tergitol 15-S-9, marketed by Union Carbide Corporation, Neodol 45-9, Neodol 23-6.5, Neodol 45-7, and Neodol 45-4, marketed by Shell Chemical Company, and Kyro EOB, marketed by The Procter & Gamble Company. (Tergitol, Neodol and Kyro are Registered Trade Marks of Union Carbide Corporation, Shell Chemical Company and The Procter & Gamble Company respectively).

(3) The condensation products of ethylene oxide with a hydrophobic base formed by the condensation of propylene oxide with propylene glycol. The hydrophobic portion of these compounds has a molecular weight of from 1500 to 1800 and exhibits water insolubility. The addition of polyoxyethylene moieties to this hydrophobic portion tends to increase the water solubility of the molecule as a whole, and the liquid character of the product is retained up to the point where the polyoxyethylene content is 50% of the total weight of the condensation product, which corresponds to condensation with up to about 40 moles of

ethylene oxide. Examples of compounds of this type include certain of the commercially available Pluronic surfactants, marketed by Wyandotte Chemical Corporation.

(4) The condensation products of ethylene oxide with the product resulting from the reaction of propylene oxide and ethylenediamine. The hydrophobic moiety of these products consists of the reaction product of ethylenediamine and excess propylene oxide, said moiety having a molecular weight of from 2500 to 3000. This hydrophobic moiety is condensed with ethylene oxide to the extent that the condensation product contains from 40% to 80% by weight of polyoxyethylene and has a molecular weight of from 5,000 to 11,000. Examples of this type of nonionic surfactant include certain of the commercially available Tetronic (Registered Trade Mark) compounds, marketed by Wyandotte Chemical Corporation.

(5) Semi-polar nonionic surfactants include water-soluble amine oxides containing one alkyl moiety of from 10 to 18 carbon atoms and 2 moieties selected from alkyl groups and hydroxyalkyl groups containing from 1 to 3 carbon atoms; water-soluble phosphine oxides containing one alkyl moiety of 10 to 18 carbon atoms and 2 moieties selected from alkyl groups and hydroxyalkyl groups containing from 1 to 3 carbon atoms; and water-soluble sulfoxides containing one alkyl moiety of from 10 to 18 carbon atoms and a moiety selected from alkyl and hydroxyalkyl moieties of from 1 to 3 carbon atoms.

As with the debonding agents that are nonionic surfactants, it is believed that essentially any anionic surfactant that is applied to the pulp sheet will render it easily fiberizable. Nonlimiting examples of suitable anionic surfactants are as follows:

(1) Water-soluble salts of the higher fatty acids, i.e., "soaps", are suitable anionic surfactants. This class of surfactants includes ordinary alkali metal soaps such as the sodium, potassium, ammonium and alkanolammonium salts of higher fatty acids containing from 8 to 24 carbon atoms and preferably from 10 to 20 carbon atoms. Soaps can be made by direct saponification of fats and oils or by the neutralization of free fatty acids.

(2) Water-soluble salts, particularly the alkali metal, ammonium and alkanolammonium salts of organic sulfuric reaction products having in their molecular structure an alkyl or alkaryl group containing from 8 to 22, especially from 10 to 20 carbon atoms and a sulfonic acid or sulfuric acid ester group. (Included in the term "alkyl" is the alkyl portion of acyl groups). Examples of this group of synthetic surfactants are the sodium and potassium alkyl sulfates, especially those obtained by sulfating the higher alcohols ($C_8$—$C_{18}$) carbon atoms produced by reducing the glycerides of tallow or coconut oil and sodium and potassium alkyl benzene sulfonates, in which the alkyl group contains from 9 to 15 carbon atoms, in straight chain or branched chain configuration, e.g., those of the type described in U.S. Pat. Nos. 2,220,099 and 2,477,383. Especially valuable, due to their commercial availability, are linear straight chain alkyl benzene sulfonates in which the average of the alkyl group is 11.8 carbon atoms, abbreviated as $C_{11.8}$ LAS.

(3) Other anionic surfactants herein include the sodium alkyl glyceryl ether sulfonates, especially those ethers of higher alcohols derived from tallow and coconut oil; sodium coconut oil fatty acid monoglyceride sulfonates and sulfates; and sodium or potassium salts of alkyl phenol ethylene oxide ether sulfate containing 1 to 10 units of ethylene oxide per molecule and wherein the alkyl groups contain 8 to 12 carbon atoms.

Other useful anionic surfactants herein include the water-soluble salts of esters of sulfonated fatty acids containing from 6 to 20 carbon atoms in the fatty acid group and from 1 to 10 carbon atoms in the ester group; water-soluble salts of 2-acyloxy-alkane-1-sulfonic acids containing from 2 to 9 carbon atoms in the acyl group and from 9 to 23 carbon atoms in the alkane moiety; alkyl ether sulfates containing from 10 to 20 carbon atoms in the alkyl group and from 1 to 30 moles of ethylene oxide; water-soluble salts of olefin sulfonates containing from 12 to 24 carbon atoms; water-soluble salts of paraffin sulfonates containing from 8 to 24 and β-alkyloxy alkane sulfonates containing from 1 to 3 carbon atoms in the alkyl group and from 8 to 20 carbon atoms in the alkane moiety.

The following examples are given to illustrate the parameters of and the process within the invention. All percentages are by weight unless otherwise indicated.

Example 1

Pulp sheet was made from fully bleached sulfate pulp on a conventional machine of the Fourdrinier type. The dried pulp sheet contained about 6% of moisture.

A debonding agent solution consisting of 10% by weight Berocell 579$^R$, a quaternary ammonium debonding agent, and 90% water was made. This solution was applied to the pulp sheet with an offset gravure printer such that the dried pulp sheet contained 0.5% by weight of debonding agent solution. The pulp sheet was then stored at room temperature for at least a week.

The pulp sheet was then fiberized. A five gram sample of the fiberized pulp was then utilized to determine the percent disintegration, i.e., the amount of the pulp that had been adequately fiberized. This was accomplished by placing the five gram sample over a 14 mesh screen and then injecting air to circulate the sample while simultaneously drawing the sample through the mesh screen with a vacuum for three minutes. The final weight of the fiberized pulp remaining on the screen was measured and then the percent disintegration was calculated as follows:

$$\%\ \text{Disintegration} = \frac{(5\ \text{grams} - \text{Final Weight})}{(5\ \text{grams})} \times 100$$

The entire above procedure was repeated several times with the level of debonding agent being applied to the pulp sheet being varied each time.

The results were as follows:

| % Debonding agent solution | % Disintegration |
|---|---|
| 0 (untreated) | 68.7 |
| 0.5 | 89.8 |
| 1.5 | 90.7 |
| 3.5 | 83.9 |

The data indicate that the addition of the debonding agent to a dried pulp sheet resulted in a substantial increase in the percent disintegration as compared to untreated pulp sheet.

Example II

The entire procedure of Example I was repeated, however, the debonding agent solution was applied to the pulp sheet with an airless sprayer. Such sprayer applied the debonding agent solution over the entire surface of the pulp sheet. This procedure was repeated several times with the level of debonding agent applied to the pulp sheet surface being varied each time. The results were as follows:

| % Debonding agent solution | % Disintegration |
|---|---|
| 0 (untreated) | 63.1 |
| 0.6 | 72.2 |
| 1.5 | 73.4 |
| 2.0 | 76.5 |

Data indicate that the addition of the debonding agent to a dried pulp sheet resulted in a substantial increase in the % Disintegration as compared to untreated pulp sheet.

Example III

Pulp sheet was produced just as that of Example 1. A debonding agent solution was made consisting of 15% by weight Berocell 579® and 85% by weight water. This solution was then sprayed onto the pulp sheet with an aerosol sprayer. An electronic balance was utilized to monitor the amount sprayed. The total level of solution added to the pulp sheet was about 6.8% by weight of the dry pulp sheet. This resulted in a Berocell 579 addition of level 1% by weight of the dry pulp sheet, referred to as Percent Addition.

A Mullenburst tester was used to measure the bursting index of the treated pulp sheets. Such test measures the tensile strength of the pulp sheet. The test was carried out on the top and then the bottom side of the pulp sheet. The average of such measurements was then calculated and is referred to as Average Mullenburst Strength.

The entire above procedure was then repeated without the debonding agent solution, with just water and with several different debonding agents. The results were as follows:

| Debonding agent | Percent addition | Average mullenburst strength |
|---|---|---|
| Berocell 579 | 1 | 158 |
| None | 0 | 207 |
| Water | 5.8 | 189 |
| Triton X-114 (nonionic surfactant) | 1 | 148 |
| Pegosperse 200-ML (nonionic surfactant) | 1 | 123 |
| Gafac RS-410 (anionic surfactant) | 1 | 158 |

The average Mullenburst strength directly indicates the ease of disintegration of a pulp sheet. It can be seen from the data that the addition of the debonding agent to the pulp sheet substantially lowers the tensile strength of the pulp sheet which thereby renders it more easily fiberizable.

**Claims**

1. A process of making fluffed fibrous pulp sheets that contain debonding agents comprising the steps of:
(a) forming said pulpsheet; and
(b) drying said pulp sheet and applying a debonding agent selected from cationic, nonionic and anionic surfactants and mixtures thereof to said pulp sheet, in an amount of from 0.001% to 5.0% based on the dry weight of the pulp sheet, after the commencement of said drying step, wherein the process comprises:
1) applying said debonding agent uniformly to the surface of said pulp sheet after the sheet has been dried to a moisture content of from 5% to 15% by weight of the sheet,
2) storing said treated sheet to permit said debonding agent to diffuse uniformly through said pulp sheet before said sheet is fiberized.

2. A process according to claim 1 wherein the debonding agent is an aqueous solution of a cationic surfactant, the solution is sprayed on to the sheet and the treated sheet is held for at least one week before fiberization.

**Patentansprüche**

1. Verfahren zur Herstellung von flockigen faserigen Pulpebahnen, die Entbindemittel enthalten, umfassend die folgenden Stufen:
(a) Herstellung der Pulpebahn und
(b) Trocknen dieser Pulpebahn und Aufbringen eines Entbindemittels ausgewählt aus kationischen, nichtionischen und anionischen Tensiden und Gemischen davon auf diese Pulpebahn in einer Menge von 0,001% bis 5,0%, bezogen auf das Trockengewicht der Pulpebahn nach Beginn dieser Trocknungsstufe, wobei das Verfahren umfaßt:
1) gleichmäßiges Aufbringen dieses Entbindemittels auf die Oberfläche dieser Pulpebahn, nachdem die Bahn auf einen Feuchtigkeitsgehalt von 5 bis 15 Gew.-% der Bahn getrocknet worden ist,
2) Lagern dieser behandelten Bahn so, daß das Entbindemittel gleichmäßig durch die Pulpebahn diffundieren kann, bevor die Bahn zerfasert wird.

2. Verfahren nach Anspruch 1, worin das Entbindemittel eine wäßrige Lösung eines kationischen Tensids ist, die Lösung auf die Bahn gesprüht wird und vor der Zerfaserung die behandelte Bahn mindestens eine Woche gelagert wird.

**Revendications**

1. Procédé de fabrication de feuilles de pâte de cellulose fibreuses et duveteuses contenant des agents déliants, comprenant les étapes qui consistent:
(a) à former ladite feuille de pâte; et
(b) à sécher ladite feuille de pâte et appliquer un agent déliant choisi parmi les tensio-actifs

cationiques, non ioniques et anioniques et leurs mélanges, à ladite feuille de pâte, en une quantité de 0,001% à 5,0%, par rapport au poids sec de la feuille de pâte, après le commencement de ladite étape de séchage, procédé qui comprend:

1) l'application uniforme dudit agent déliant sur la surface de ladite feuille de pâte après que la feuille ait été séchée jusqu'à une teneur en humidité de 5% à 15% en poids de la feuille,
2) le stockage de ladite feuille traitée pour permettre audit agent déliant de diffuser uniformément à travers ladite feuille de pâte avant le défibrage de ladite feuille.

2. Procédé selon la revendication 1, dans lequel l'agent déliant est une solution aqueuse d'un tensio-actif cationique, la solution est pulvérisée sur la feuille et la feuille traitée est maintenue pendant au moins une semaine avant le défibrage.